# EUROPÄISCHE PATENTSCHRIFT

(11) **EP 0 549 601 B1**
(45) Veröffentlichungstag und Bekanntmachung des Hinweises auf die Patenterteilung: **26.04.1995**
(21) Anmeldenummer: 91914582.1
(22) Anmeldetag: 08.08.1991
(51) Int. Cl.: C07C 19/08, C07C 17/20

(54) **VERFAHREN ZUR HERSTELLUNG VON WEITGEHEND FLUORIERTEN ALKYLBROMIDEN**
METHOD OF PREPARING EXTENSIVELY FLUORINATED ALKYL BROMIDES
PROCEDE DE PRODUCTION DE BROMURES D'ALKYLE TRES FLUORES

(30) Priorität: 01.09.1990 DE 4027766
(43) Veröffentlichungstag der Anmeldung: 07.07.1993
(73) Patentinhaber: RIEDEL-DE HAEN AKTIENGESELLSCHAFT, D-30926 Seelze (DE)
(72) Erfinder: WINTERFELDT, Andreas, D-3013 Barsinghausen 1 (DE); BARTELS, Günter, D-3006 Grossburgwedel (DE); KNIEPS, Reinhard, D-3000 Hannover 91 (DE)
(74) Vertreter: Muley, Ralf, Dr.
(86) Internationale Anmeldenummer: EP9101502
(87) Internationale Veröffentlichungsnummer: WO9204307

(56) Entgegenhaltungen:
- HOUBEN-WEYL, "Methoden der organischen Chemie, Band V/4, Halogenverbindungen", Georg Thieme Verlag, Stuttgart, DE, 1960, S. 354-360
- CHEMICAL ABSTRACTS, Band 104, 1986, S. 619, Zusammenfassung Nr. 88 106p. Columbus, Ohio, USA; Y. FURUTAKA et al: "Perfluoroalkyl bromides"; & JP-A-60 184 033
- JOURNAL OF THE AMERICAN CHEMICAL SOCIETY, Band 79, 5. August 1957, Easton, S. 4170-4174; A. H. FAINSBERG et al: "Preferential replacement reactions of highly fluorinated alkyl halides. II. Some reactions of fluorinated allyl iodides"

## Beschreibung

Die vorliegende Erfindung betrifft ein Verfahren zur Herstellung von weitgehend fluorierten Alkylbromiden, insbesondere von Perfluoralkylbromiden, ausgehend von weitgehend fluorierten Alkyliodiden, insbesondere von Perfluoralkyliodiden.

Weitgehend fluorierte Alkylbromide, insbesondere Perfluoralkylbromide werden, z.B. als Zwischenprodukte für die Herstellung von polymeren Flüssigkeiten, Harzen und Elastomeren, als Röntgenkontrastmittel, zur Herstellung von Pharmazeutischen Präparaten und in wäßriger Emulsion als Blutersatzstoff verwendet. Ein als Blutersatzstoff bevorzugtes Perfluoralkylbromid ist das Perfluoroctylbromid.

Für die Herstellung von Perfluoralkylbromiden sind bereits eine Reihe von Verfahren bekannt. So werden z.B. gemäß der japanischen Patentschrift JP 60 184 033 (C.A. Vol 104 (1986), 88106p) Perfluoralkyliodide in Anwesenheit von Radikalinitiatoren mit elementarem Brom zu Perfluoralkylbromiden umgesetzt. Hazeldine (J. Chem. Soc. 1953, 3761 - 3768) beschreibt auf der Seite 3763 und 3766 die Umsetzung von Perfluoralkyliodiden mit elementarem Brom unter Bestrahlung mit UV-Licht. Beide Verfahrensweisen sind durch den Einsatz von elementarem Brom, durch das Freisetzen von elementaren Iod, Interhalogenverbindungen sowie Fluorwasserstoff mit erheblichen Werkstoff- und sicherheitstechnischen Problemen verbunden.

Weitere Herstellungsverfahren für Perfluoralkylbromide sind z.B. (R_{F}= Perfluoralkyl): Die Einwirkung von Brom auf Verbindungen R_{F} - SF₅ bei 500°C in Gegenwart von Nickel (USP-3456024); die Einwirkung von Brom auf Verbindungen R_{F} -SO₂Na in Gegenwart von KI/I₂ (C.A., Vol. 107 (1987), 236043); die Einwirkung von Brom auf Salze von perfluorierten Carbonsäuren (USP-2678953), insbesondere auf R_{F}COOAg (USP-2678953 und Hauptschein et/al., J. Am. Chem. Soc. 74 (1952), 1347ff); die Einwirkung von Brom auf Verbindungen R_{F}H bei gleichzeitiger Bestrahlung mit UV-Licht (J. Chem. Soc. 1953, 3761). Bei all diesen Verfahren treten durch die Verwendung von elementarem Brom erhebliche Werkstoff-und sicherheitstechnische Probleme auf. Darüberhinaus Sind die Ausgangsverbindungen schwer zugänglich oder müssen über eine zusätzliche Verfahrensstufe aus den entsprechenden Perfluoralkyliodiden hergestellt werden. Dies gilt auch für die Herstellung von Perfluoralkylbromiden durch Umsetzung von R_{F}SO₃Cl mit HBr-Gas in Gegenwart eines Katalysators bei 125°C. (EP-A1-0298870).

Nach Fainberg et/al. JACS 79. 4172 (1957) kann Perfluoralkylbromid durch Umsetzung von Perfluoralkyliodid mit Lithiumbromid in Aceton hergestellt werden. Die naheliegende Übertragung dieser Umhalogenierung auf andere Perfluoralkyliodide gelingt aber nicht, weil in normalen Perfluoralkyliodiden keine Aktivierung des Iods durch eine Allylgruppe vorliegt. Wie aus dem nachfolgenden Vergleichsbeispiel 1 hervorgeht, sind die von Fainberg et/al. beschriebenen Reaktionsbedingungen auf Perfluoroctyliodid nicht erfolgreich übertragbar. Das nachfolgende Vergleichsbeispiel 2 zeigt, daß auch der Versuch einer Reaktionsbeschleunigung unter Phasentransfer-Katalyse zu keinem befriedigenden Ergebnis führt.

Die Vergleichsbeispiele entsprechen in ihrem Ergebnis den Erwartungen, da bekannt ist, daß Fluoratome die Reaktivität von Alkylhalogeniden in nucleophilen Austausch-Reaktionen deutlich herabsetzen, (vgl. z.B. Houben-Weyl, Methoden der organischen Chemie, 4. Aufl., Bd. 5/4, S. 685, 688).

Die bisher bekannten Verfahren zur Herstellung weitgehend fluorierter Alkylbromide, insbesondere von Perfluoralkylbromiden sind aus den vorstehend genannten Gründen nicht befriedigend. Es ist daher Aufgabe der vorliegenden Erfindung, ein technisch einfaches Verfahren zur Herstellung von weitgehend fluorierten Alkylbromiden, insbesondere von Perfluoralkylbromiden, ausgehend von den leicht zugänglichen weitgehend fluorierten Alkyliodiden, insbesondere von Perfluoralkyliodiden, zur Verfügung zu stellen.

Diese Aufgabe wird gelöst durch ein Verfahren zur Herstellung von fluorierten Alkylbromiden der Formel I

X-CₙF₂ₙ-Br (I)

worin X = H, F oder (F₃C)₂CF- und n = 1 bis 20 bedeuten, ausgehend von fluorierten Alkyliodiden der Formel III

X-CₙF₂ₙ-I (III)

worin X und n wie oben definiert sind; dadurch gekennzeichnet, daß ein fluoriertes Alkyliodid der Formel III mit einer organischen Bromverbindung, in der das Bromatom kovalent an ein Kohlenstoffatom oder ein Stickstoffatom gebunden ist, umgesetzt wird.

In den Formeln I und III bedeutet n vorzugsweise 4 bis 16 und besonders bevorzugt 6 bis 12.

In den Formeln I und III besitzt die Gruppe -CₙF₂ₙ- insbesondere die Form

-(CF₂)ₙ- (II)

In den Formeln I und III bedeutet X vorzugsweise (F₃C)₂-CF- und besonders bevorzugt F. Das erfindungsgemäße Verfahren ist daher besonders zur Herstellung von Perfluoralkylbromiden, besonders bevorzugt solcher mit 6 bis 12 C Atomen, ganz besonders bevorzugt zur Herstellung von Perfluoroctylbromid, geeignet. Das heißt, eine ganz besonders bevorzugte Ausgangsverbindung ist Perfluoroctyliodid.

Die als Ausgangsverbindungen benutzten fluorierten Alkyliodide der Formel III sind bekannt und/oder lassen sich nach verschiedenen für diese Verbindungsklasse bekannten Verfahren herstellen.

Organische Bromverbindungen, in denen das Bromatom kovalent an ein Kohlenstoffatom oder ein Stickstoffatom gebunden ist, sind beispielsweise Alkylbromide, vorzugsweise hochbromierte Alkylbromide, insbesondere solche mit 1 bis 6 Kohlenstoffatomen; Säurebromide, insbesondere solche der Alkylcarbonsäuren mit 1 bis 6 Kohlenstoffatomen oder der Phenylcarbonsäuren; N-bromierte Amide, insbesondere solche der Alkylcarbonsäuren mit 1 bis 6 Kohlenstoffatomen oder der Phenylcarbonsäuren.

Bei der Auswahl einer geeigneten organischen Bromverbindung ist vorteilhafterweise deren Siedepunkt zu berücksichtigen, um in der Reaktionsmischung eine für die jeweilige Synthese ausreichend hohe Reaktionstemperatur zu erreichen.

Darüber hinaus sind solche organischen Bromverbindungen besonders vorteilhaft, die möglichst wenige oder besser keine radikalisch leicht spaltbaren Kohlenstoff-Wasserstoff-Bindungen enthalten, da diese zu unerwünschten Nebenreaktionen, wie beispielsweise die Reduktion des eingesetzten Perfluoralkyliodids, führen können.

Bevorzugte organische Bromverbindungen sind Bromoform, Dibrommethan, N-Bromsuccinimid, Benzoylbromid, 2-Bromisobuttersäurebromid und Bromfluorbenzoylbromid. Besonders bevorzugt ist Tetrabrommethan.

Die genannten organischen Bromverbindungen sind bekannt und/oder lassen sich nach verschiedenen für diese Verbindungsklasse bekannten Verfahren herstellen.

Die erfindungsgemäße Umsetzung wird durch einfaches Vermischen des fluorierten Alkyliodids der Formel III mit der organischen Bromverbindung und gegebenenfalls Erhitzen der Mischung durchgeführt. Der Zusatz eines Lösungsmittels ist nicht erforderlich. Die Umsetzung kann jedoch auch in einem geeigneten Lösungsmittel oder Lösungsmittelgemisch durchgeführt werden.

Pro Mol fluoriertem Alkyliodid der Formel III wird soviel organische Bromverbindung eingesetzt, daß ein ausreichender Umsatz erzielt wird. Vorzugsweise werden pro Mol fluoriertem Alkyliodid der Formel III 0,4 bis 4 mol, vorzugsweise 0,5 bis 2 mol organische Bromverbindung eingesetzt.

Pro Mol fluoriertem Alkyliodid der Formel III können auch mehr als 4 mol organische Bromverbindung eingesetzt werden. Dies bringt aber keine Vorteile. Bei zu geringen Mengen an organischer Bromverbindung wird nur ein ungenügender Umsatz erzielt.

Die Reaktionstemperatur liegt vorzugsweise zwischen 0°C und dem Siedepunkt des Reaktionsgemisches unter Normaldruck. Die Reaktion wird insbesondere bei einer Temperatur von 20°C bis zum Siedepunkt des Reaktionsgemisches unter Normaldruck, vorzugsweise bis zum Siedepunkt des fluorierten Alkyliodids der Formel III unter Normaldruck, durchgeführt. In vielen Fällen wird die Umsetzung bei Temperaturen von 50 bis 160°C durchgeführt. Die Umsetzungsgeschwindigkeit ist, wie üblich, bei höheren Temperaturen größer als bei niederen.

Es kann zweckmäßig sein, die Reaktion unter Inertgasatmosphäre, z.B. unter Argon, durchzuführen.

Falls ein Lösungsmittel bzw. Lösungsmittelgemisch verwendet werden soll, so sind dazu alle inerten Lösungsmittel, die eine genügend hohe Reaktionstemperatur erlauben, geeignet. Bevorzugte Lösungsmittel sind Fluorbenzol, Dibromfluorbenzol, Nitrobenzol und Biphenyl.

Die Umsetzung bzw. Aufarbeitung kann auf verschiedene Weise erfolgen und z.B. so durchgeführt werden, daß gebildetes fluoriertes Alkylbromid der Formel I während der Reaktion oder nach der Reaktion abdestilliert wird. Man kann auch vorzugsweise so aufarbeiten, daß nach der Umsetzung die Temperatur des Reaktionsgemisches gegebenenfalls erniedrigt und das Reaktionsgemisch mit Wasser versetzt, d.h. durchmischt wird, und danach die entstandenen Phasen getrennt werden und diese destillativ in ihre Bestandteile, d.h. insbesondere in fluoriertes Alkylbromid der Formel I und nicht umgesetztes fluoriertes Alkyliodid der Formel III und organische Bromverbindung zerlegt wird. Die bei dieser Aufarbeitung zurückgewonnenen Ausgansprodukte können der Reaktion von neuem zugeführt werden.

Bei dem erfindungsgemäßen Verfahren werden die gewünschten fluorierten Alkylbromide der Formel I, vorzugsweise die Perfluoralkylbromide, in Ausbeuten bis über 90 % (bezogen auf umgesetztes Ausgangsprodukt) erhalten. Die nicht umgesetzten Ausgangsprodukte können auf einfache Weise zurückgewonnen und von neuem eingesetzt werden.

Die gaschromatographisch bestimmten Reinheiten der Endprodukte sind hoch und liegen in vielen Fällen über 99 %.

Anhand der nachstehenden Beispiele wird die Erfindung weiter erläutert:

### Beispiel 1

640 g Perfluoroctyliodid (1,17 Mol), 776 g Tetrabrommethan (2,35 Mol) in 520 g Dibromfluorbenzol werden 72 Stunden unter Rückfluß gerührt. Man läßt auf 80°C abkühlen und gibt 2 g Natriumsulfit in 100 g Wasser zu und rührt 30 min bei 60°C. Anschließend werden in dem entstandenen 3-Phasensystem die Phasen getrennt. Die untere Dibromfluorbenzol/Tetrahalogenmethan enthaltende Phase kann nach Wasserdampfdestillation wieder eingesetzt werden.

Die mittlere Perfluoroctyliodid/Perfluoroctylbromid enthaltende Phase wird zweimal mit 100 g Essigsäure extrahiert und danach fraktioniert destilliert. Die obere wäßrige Phase wird verworfen.
- Ausbeute:: 260 g PFOB entspr. 91 % bezogen auf umgesetzes PFOI
330 g PFOI (wird wiederverwendet)
- Gehalt (GC):: größer 99 %
- Kp.:: 142 - 143 °C

### Beispiel 2

20 g Perfluoroctyliodid (36,6 mMol) und 20,7 g 3-Brom-4-fluorbenzoylbromid (73,3 mMol) werden 24 Stunden bei 150°C gerührt. Nach Abkühlen auf 40 °C wird mit 100 g Wasser versetzt und 10 g Na₂CO₃ und 1 g Natriumsulfit zugesetzt. Nach Phasentrennung wird die organische Phase fraktioniert destilliert.
- Ausbeute:: 3 g PFOB entspr. 81 % auf umgesetztes PFOI
16 g PFOI (wird wiederverwendet)
- Gehalt:: größer 99 %
- Kp.:: 142 - 143 °C

### Beispiel 3

640 g Perfluoroctyliodid (1,17 Mol), 776 g Tetrabrommethan (2,35 Mol) in 400 g Fluorbenzol werden zum Rückfluß erhitzt. Bis zu einer Sumpftemperatur von 150°C wird über eine 30 cm Vigreux-Kolonne "Fluorbenzol" abdestilliert. Bei 150°C Sumpftemperatur wird 72 Stunden unter Rühren gekocht. Nach Reaktionsende läßt man auf 70°C abkühlen und setzt das anfangs abdestillierte "Fluorbenzol" wieder zu. Unter Rühren werden dann 200 g Natriumdisulfit in 500 g Wasser zudosiert uni danach 30 Minuten bei Raumtemperatur gerührt.

Anschließend werden in dem entstandenen 3-Phasensystem die Phasen getrennt.

Die untere Fluorbenzol/Tetrallalogenmethan enthaltene Phase kann nach Regeneration wieder eingesetzt werden.

Die mittlere PFOI/PFOB enthaltene Phase wird zweimal mit 100 g Ameisensäure extrahiert, mit Wasser gewaschen und danach fraktioniert destilliert.

Die obere wäßrige Phase wird verworfen.
- Ausbeute:: 215 g PFOB entspr. 88 % bezogen auf umgesetztes PFOI
373 g PFOI (wird wiederverwendet)
- Gehalt (GC):: größer 99 %
- Kp.:: 142 - 143 °C

### Beispiel 4

20 g PFOI (36,6 mMol) und 16,9 g Bromisobuttersäurebromid (73,3 mMol) werden 72 Stunden bei einer Sumpftemperatur von anfangs 150°C am Rückfluß gekocht. Nach Abkühlen auf 40°C wird mit 100 g Wasser versetzt und 10 g Na₂CO₃ sowie 1 g Natriumsulfit zugesetzt. Nach Phasentrennung wird die organische Phase fraktioniert destilliert.
- Ausbeute:: 3 g PFOB entspr. 81 % auf umgesetztes PFOI
16 g PFOI (wird wiederverwendet)
- Gehalt:: größer 99 %
- Kp.:: 192 - 193 °C

### Beispiel 5

50 g PFOI (91,6 mMol), 16,3 g (91,6 mMol) N-Bromsuccinimid werden in 100 g Dibromfluorbenzol 24 Stunden bei 120°C gerührt. Nach Abkühlen auf 70°C werden 5 g Natriumdisulfit in 50 g Wasser zugetropft. Nach Phasentrennung wird die isolierte PFOB/PFOI-Phase mit Wasser gewaschen und danach fraktioniert destilliert.
- Ausbeute:: 4 g PFOB entspr. 48 % bezogen auf umgesetztes PFOI
41 g PFOI (wird wiederverwendet)

### Vergleichsbeispiel 1

Eine lösung von 19 g LiBr (220 mMol) in 150 ml trockenen Aceton wird innerhalb 10 Minuten mit 100 g Perfluoroctyliodid (183 mMol) versetzt und danach 8 Stunden am Rückfluß gekocht. Man gießt in 500 ml Wasser, trennt die organische Phase ab und trocknet mit wenig CaCl₂.
- Ausbeute:: 100 g Perfluoroctyliodid 98%ig.

Perfluoroctylbromid ist gaschromatographisch nicht nachweisbar.

### Vergleichsbeispiel 2

100 g Perfluoroctyliodid (183 mMol), 75 g CaBr₂ (375 mMol), 25 ml Wasser und 1 g Tetrabutylammoniumbromid (1,5 Mol %) werden 5 Stunden am Rückfluß gekocht. Man gießt in 500 ml Wasser, trennt die organische Phase ab, wäscht die organische Phase halogenidfrei und trocknet mit wenig CaCl₂.
- Ausbeute:: 94 g
- GC:: 1 % Perfluoroctylbromid
96 % Perfluoroctyliodid

Analog Vergleichsbeispiel 2 erhalt man mit 1 g Tetrabutylphosphoniumbromid (1,5 Mol%) als Phasentransferkatalysator:
- Ausbeute:: 95 g
- GC:: 1 % Perfluoroctylbromid
97 % Perfluoroctyljodid

## Patentansprüche

1. Verfahren zur Herstellung von fluorierten Alkylbromiden der Formel I
X-CₙF₂ₙ-Br (I)
worin X = H, F oder (F₃C)₂CF- und n = 1 bis 20 bedeuten, ausgehend von fluorierten Alkyliodiden der Formel III
X-CₙF₂ₙ-I (III)
worin X und n wie oben definiert sind; dadurch gekennzeichnet, daß ein fluoriertes Alkyliodid der Formel III mit einer organischen Bromverbindung, in der das Bromatom kovalent an ein Kohlenstoffatom oder ein Stickstoffatom gebunden ist, umgesetzt wird.

2. Verfahren nach Anspruch 1, dadurch gekennzeichnet, daß n 4 bis 16, besonders bevorzugt 6 bis 12, bedeutet.

3. Verfahren nach Anspruch 1 und/oder 2, dadurch gekennzeichnet, daß in den Formeln I und III die Gruppe -CₙF₂ₙ- die Form -(CF₂)ₙ- besitzt und/oder X die Bedeutung (F₃C)₂CF-, vorzugsweise F, besitzt.

4. Verfahren nach einem oder mehreren der Ansprüche 1 bis 3, dadurch gekennzeichnet, daß als fluoriertes Alkyliodid der Formel III Perfluoroctyliodid eingesetzt wird.

5. Verfahren nach einem oder mehreren der Ansprüche 1 bis 4, dadurch gekennzeichnet, daß als organische Bromverbindungen, in denen das Bromatom kovalent an ein Kohlenstoffatom oder ein Stickstoffatom gebunden ist, Alkylbromide, vorzugsweise hochbromierte Alkylbromide, insbesondere solche mit 1 bis 6 Kohlenstoffatomen; Säurebromide, insbesondere solche der Alkylcarbonsäuren mit 1 bis 6 Kohlenstoffatomen oder der Phenylcarbonsäuren; N-bromierte Amide, insbesondere solche der Alkylcarbonsäuren mit 1 bis 6 Kohlenstoffatomen oder der Phenylcarbonsäuren, eingesetzt werden.

6. Verfahren nach einem oder mehreren der Ansprüche 1 bis 5, dadurch gekennzeichnet, daß als organische Bromverbindungen, in denen das Bromatom kovalent an ein Kohlenstoffatom oder ein Stickstoffatom gebunden ist, Tetrabrommethan eingesetzt wird.

7. Verfahren nach einem oder mehreren der Ansprüche 1 bis 6, dadurch gekennzeichnet, daß das fluorierte Alkyliodid der Formel III und die organische Bromverbindung im Molverhältnis 1 : (0,4 bis 4), vorzugsweise 1 : (0,5 bis 2,0) eingesetzt werden.

8. Verfahren nach einem oder mehreren der Ansprüche 1 bis 7, dadurch gekennzeichnet, daß die Umsetzung bei einer Temperatur von 20°C bis zum Siedepunkt des Reaktionsgemisches, vorzugsweise bei einer Temperatur von 50 bis 160°C, durchgeführt wird.

9. Verfahren nach einem oder mehreren der Ansprüche 1 bis 8, dadurch gekennzeichnet, daß nach beendeter Umsetzung das Reaktionsgemisch mit Wasser vermischt wird und das Gemisch dann in die entstandenen Phasen getrennt wird und daß dann diese destillativ in ihre Bestandteile zerlegt werden.

10. Verfahren nach einem oder mehreren der Ansprüche 1 bis 9, dadurch gekennzeichnet, daß bei der Aufarbeitung zurückgewonnenes, fluoriertes Alkyliodid der Formel III von neuem als Ausgangsprodukt eingesetzt wird.

## Claims

1. Process for the preparation of fluorinated alkyl bromides of the formula I
X-CₙF₂ₙ-Br (I)
in which X is H, F or (F₃C)₂CF and n is 1 to 20 starting from fluorinated alkyl iodides of the formula III
X-CₙF₂ₙ-I (III)
wherein X and n are defined as above; characterised in that a fluorinated alkyl iodide of the formula III is reacted with an organic bromine compound in which the bromine atom is bound covalently to a carbon atom or a nitrogen atom.

2. Process according to Claim 1, characterised in that n denotes 4 to 16, particularly preferably 6 to 12.

3. Process according to Claim 1 and/or 2, characterised in that in formulae I and III the group -CₙF₂ₙ- has the form -(CF₂)ₙ- and/or X has the meaning (F₃C)₂CF-, preferably F.

4. Process according to one or more or Claims 1 to 3, characterised in that perfluorooctyl iodide is used as fluorinated alkyl iodide of the formula III.

5. Process according to one or more of Claims 1 to 4, characterised in that the organic bromine compounds used, in which the bromine atom is bound covalently to a carbon atom or a nitrogen atom, are alkyl bromides, preferably highly brominated alkyl bromides, in particular those having 1 to 6 carbon atoms; acid bromides, in particular those of alkylcarboxylic acids having 1 to 6 carbon atoms or of phenylcarboxylic acids; N-brominated amides, in particular those of alkylcarboxylic acids having 1 to 6 carbon atoms or of phenylcarboxylic acids.

6. Process according to one or more of Claims 1 to 5, characterised in that tetrabromomethane is used as organic bromine compounds in which the bromine atom is bound covalently to a carbon atom or a nitrogen atom.

7. Process according to one or more of Claims 1 to 6, characterised in that the fluorinated alkyl iodide of the formula III and the organic bromine compound are used in a molar ratio of 1:(0.4 to 4), preferably 1:(0.5 to 2.0).

8. Process according to one or more of Claims 1 to 7, characterised in that the reaction in carried out at a temperature of 20° C up to the boiling point of the reaction mixture, preferably at a temperature of 50 to 160°C.

9. Process according to one or more of Claims 1 to 8, characterised in that after the reaction is complete the reaction mixture is mixed with water and the mixture is then separated into the phases formed and these are then separated into their components by distillation.

10. Process according to one or more of Claims 1 to 9, characterised in that fluorinated alkyl iodide of the formula III recovered during work-up is again used as starting material.

## Revendications

1. Procédé pour la préparation de bromures d'alkyle fluorés de formule I :
X-Cₙ-F₂ₙ-Br (I)
où X = H, F ou (F₃C)₂CF- et n = 1 à 20, en partant d'iodures d'alkyle fluorés de formule III :
X-CₙF₂ₙ-I (III)
où X et n sont définis comme ci-dessus ; caractérisé en ce qu'on fait réagir un iodure d'alkyle fluoré de formule III avec un composé organique de brome, dans lequel l'atome de brome est lié par une liaison covalente à un atome de carbone ou un à atome d'azote.

2. Procédé selon la revendication 1, caractérisé en ce que n va de 4 à 16, de manière particulièrement préférée de 6 à 12.

3. Procédé selon la revendication 1 et/ou 2, caractérisé en ce que dans les formules I et III, le groupe -CₙF₂ₙ- a la forme de -(CF₂)ₙ- et/ou X a la signification de (F₃C)₂CF-, de préférence de F.

4. Procédé selon une ou plusieurs des revendications 1 à 3, caractérisé en ce qu'on utilise en tant qu'iodure d'alkyle fluoré de formule III l'iodure de perfluorooctyle.

5. Procédé selon une ou plusieurs des revendications 1 à 4, caractérisé en ce que, en tant que composés organiques de brome dans lesquels l'atome de brome est lié par une liaison covalente à un atome de carbone ou à un atome d'azote, on utilise des bromures d'alkyle, de préférence des bromures d'alkyle très bromés, plus particulièrement ceux qui comportent de 1 à 6 atomes de carbone ; des bromures d'acides, plus particulièrement ceux des acides alkylcarboxyliques comportant de 1 à 6 atomes de carbone ou des acides phénylcarboxyliques ; des amides N-bromés, plus particulièrement ceux des acides alkylcarboxyliques comportant de 1 à 6 atomes de carbone ou des acides phénylcarboxyliques.

6. Procédé selon une ou plusieurs des revendications 1 à 5, caractérisé en ce que, en tant que composés organiques de brome dans lesquels l'atome de brome est lié par une liaison covalente à un atome de carbone ou à un atome d'azote, on utilise le tétrabromométhane.

7. Procédé selon une ou plusieurs des revendications 1 à 6, caractérisé en ce qu'on utilise l'iodure d'alkyle fluoré de formule III et le composé organique de brome dans un rapport molaire de 1:(0,4 à 4), de préférence 1:(0,5 à 2,0).

8. Procédé selon une ou plusieurs des revendications 1 à 7, caractérisé en ce qu'on met en oeuvre la réaction à une température de 20 °C jusqu'au point d'ébullition du mélange réactionnel, de préférence à une température de 50 à 160 °C.

9. Procédé selon une ou plusieurs des revendications 1 à 8, caractérisé en ce qu'après avoir terminé la réaction, on mélange le mélange réactionnel avec l'eau et on sépare ensuite le mélange en les phases formées et en ce qu'ensuite celles-ci sont fractionnées par distillation en leurs composants.

10. Procédé selon une ou plusieurs des revendications 1 à 9, caractérisé en ce que l'iodure d'alkyle fluoré de formule III, récupéré au cours du traitement complémentaire, est réutilisé en tant que produit de départ.
